# EUROPEAN PATENT APPLICATION

(11) **EP 1 918 276 A1**
(43) Date of publication of application: **07.05.2008**
(21) Application number: 07254350.7
(22) Date of filing: 02.11.2007
(51) Int. Cl.: C07D 207/16

(54) **Production method of optically active 2- [ (N-Benzylprolyl) Amino ] Benzophenone compound**

(30) Priority: 03.11.2006 US 856298 P
(71) Applicant: Ajinomoto Co., Inc., Tokyo 104-8315 (JP)
(72) Inventor: Hamada, Takayuki c/o Ajinomoto Co., Inc., Kawasaki-shi, Kanagawa 210-8681 (JP); Izawa, Kunisuke c/o Ajinomoto Co., Inc., Kawasaki-shi, Kanagawa 210-8681 (JP); Soloshonok, Vadim A. c/o Ajinomoto Co., Inc., Kawasaki-shi, Kanagawa 210-8681 (JP)
(74) Representative: Nicholls, Kathryn Margaret

(57) **Abstract**

The present invention relates to a production method of optically active 2-[(N-benzylprolyl)amino]-5-chlorobenzophenone (3) or a salt thereof, which includes reacting optically active N-benzylproline (1) with 2-amino-5-chlorobenzophenone (2) in acetonitrile and in the presence of an amide compound and thionyl chloride. According to the method of the present invention, optically active 2-[(N-benzylprolyl)amino]-5-chlorobenzophenone (3) or a salt thereof can be conveniently produced at a high purity and in a high yield: 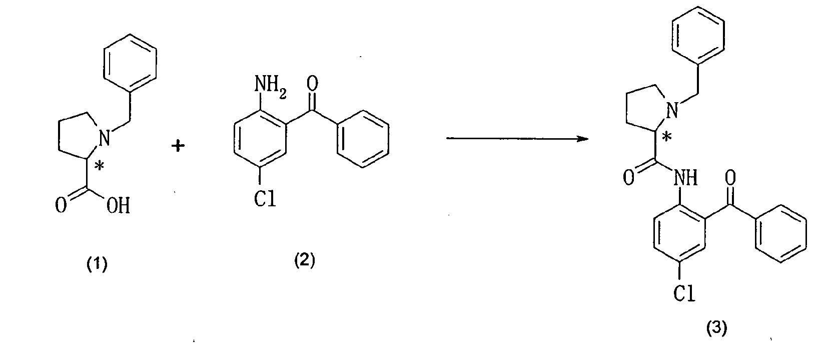 wherein * shows an asymmetric carbon atom and the configuration of the asymmetric carbon atom is S or R.

## Description

The present invention relates to a production method of an optically active 2-[(N-benzylprolyl)amino]benzophenone compound useful as a chiral auxiliary. In addition, the present invention relates to a production method of an Ni(II) complex useful as a synthetic intermediate of optically active amino acid.

(*S*)- or (R)-2-[(N-benzylprolyl)amino]benzophenone represented by the following formula (hereinafter to be also referred to as BPB) is known to be useful as a chiral auxiliary for the synthesis of an optically active amino acid.

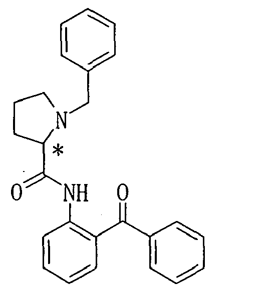

BPB is applicable to various reactions such as synthesis of amino acid and α-alkylamino acid by asymmetric alkylation reaction, synthesis of β-hydroxy-α-amino acid by asymmetric aldol reaction, synthesis of proline by asymmetric Michael addition reaction and the like.

As shown in the following scheme, for example, an Ni(II) complex of Schiff's base obtained from glycine and (S)-BPB is reacted with benzyl bromide in the presence of a base, and the obtained compound is treated with an acid to stereoselectively give (S)-phenylalanine. In addition, an Ni(II) complex of Schiff's base obtained from alanine and (S)-BPB is reacted with benzyl bromide in the presence of a base, and the obtained compound is treated with an acid to stereoselectively give α-methyl-(S)-phenylalanine. By a similar reaction using (R)-BPB, α-methyl-(R)-phenylalanine can be stereoselectively produced (J. Chem. Soc. Perkin Trans. I, 1988, 305-311).

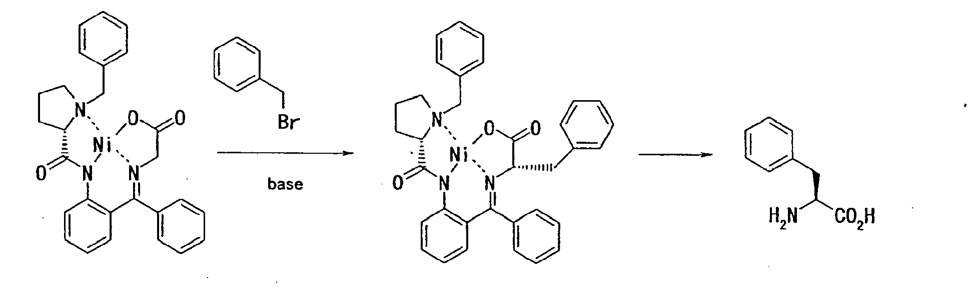

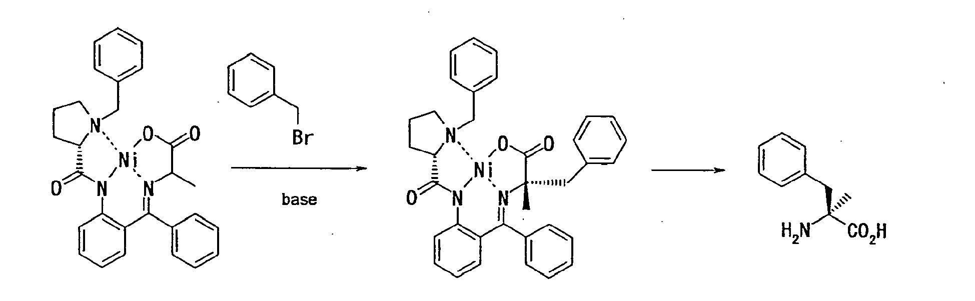

Moreover, (S)-serine can be stereoselectively produced by reacting an Ni(II) complex of Schiff's base obtained from glycine and (S)-BPB with formaldehyde in the presence of a base, and treating the obtained compound with an acid (J. Am. Chem. Soc. 1985, 107, 4252-4259).

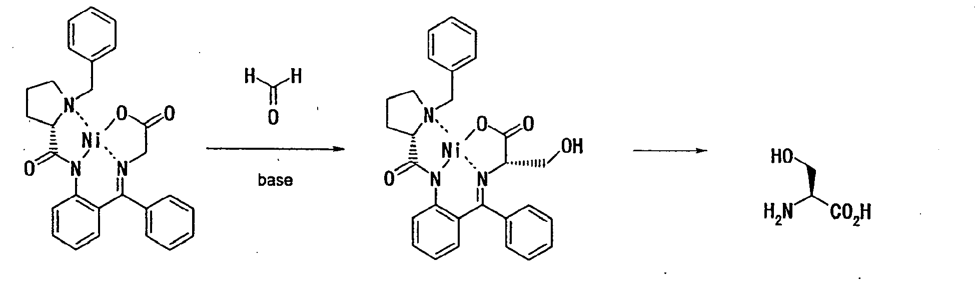

Moreover, 3-methyl-(S)-pyroglutamic acid can be stereoselectively produced by reacting an Ni(II) complex of Schiff's base obtained from glycine and (S)-BPB with N-[(E)-enoyl]-1,3-oxazolidin-2-one in the presence of a base, and treating the obtained compound with an acid (J. Am. Chem. Soc. 2005, 127, 15296-15303).

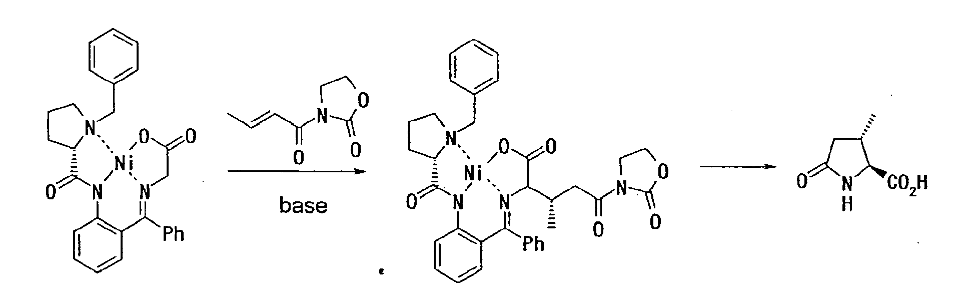

As a production method of optically active BPB, the following two methods are known.
(Method 1) Tetrahedron: Asymmetry, Vol. 9, pp. 4249-4252, 1998 discloses a method of obtaining (S)-BPB, which comprises reacting (S)-N-benzylproline (1.6 equivalents relative to 2-aminobenzophenone) with thionyl chloride at -30°C in dichloromethane, reacting the obtained acid chloride with 2-aminobenzophenone and recrystallizing the resulting compound from ethanol. However, J. Org. Chem., Vol. 68, No. 18, pp. 7104-7107, 2003 describes that recrystallization from ethanol to give BPB is not practical, and the actual yield is below 75%. Moreover, WO2005/085178 discloses a method of obtaining (R)-BPB by reacting (R)-N-benzylproline with 2-aminobenzophenone by a similar method, but the yield is 74%.

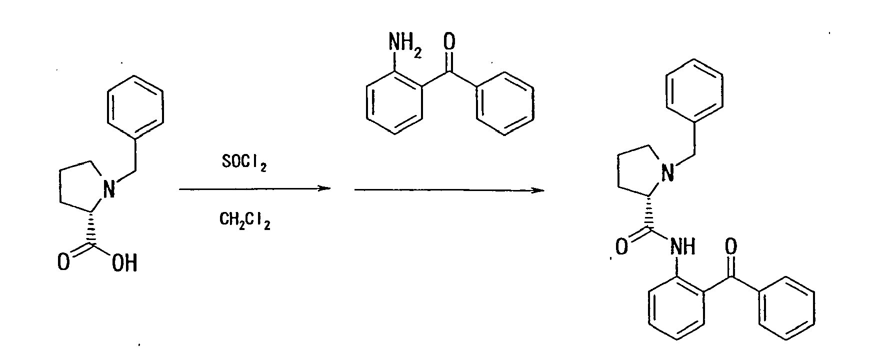

In addition, SU1447820 describes that the yield when acid chloride of (S)-N-benzylproline (1.5 equivalents relative to 2-aminobenzophenone) is reacted with 2-aminobenzophenone in dichloromethane is 85% at -30°C, 81% at -10°C, and 74% at 0°C.. When it is reacted with 2-amino-5-chlorobenzophenone instead of 2-aminobenzophenone, the yield is described to be 72% at - 30°C, 68% at -10°C and 60% at 0°C.
(Method 2) J. Org. Chem., Vol. 68, No. 18, pp. 7104-7107, 2003 discloses, as an improved method of Method 1, a method of obtaining (S)-BPB, which comprises reacting (S)-N-benzylproline (1.1 equivalents relative to 2-aminobenzophenone) with methanesulfonyl chloride (MsCl) in dichloromethane in the presence of 1-methylimidazole, and reacting the obtained mixed acid anhydride with 2-aminobenzophenone. In addition, a purification method comprising treating the obtained BPB with hydrochloric acid/acetone to give a hydrochloride of BPB is disclosed.

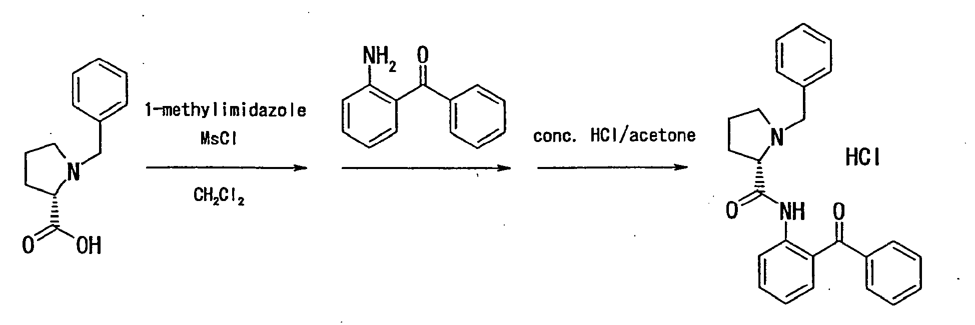

While this method is a good method for small-scale reactions in that the yield is high, since industrial large scale reactions cannot be controlled easily due to an exothermic effect produced by the addition of MsCl to a solution of N-benzylproline and 1-methylimidazole in dichloromethane. Thus, the method is not entirely an appropriate method.

According to the finding of the present inventors, optically active 2-[(N-benzylprolyl)amino]-5-chlorobenzophenone (hereinafter to be also referred to as BPC) represented by the formula (3) below, which is a derivative of BPB, is similarly useful as a chiral auxiliary, as is BPB.

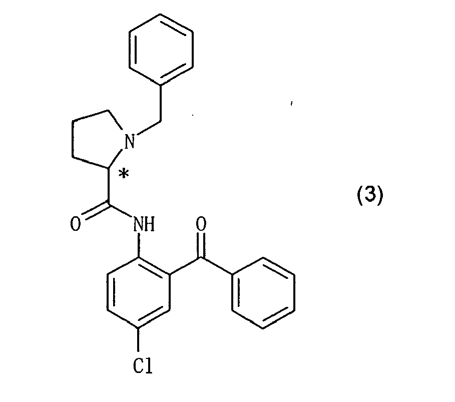

According to the finding of the present inventors, it was clarified that, when the reaction is carried out using 2-amino-5-chlorobenzophenone instead of 2-aminobenzophenone according to the above-mentioned Method 1, the objective product could not be purified by recrystallization, since it was an oil. While purification was possible through a step for extracting the objective product from the reaction mixture, a step for concentrating the extraction solvent, and a step for crystallization to give a hydrochloride, the yield of the hydrochloride crystals was about 23%. A problem also occurred in that, during cooling after addition of thionyl chloride, the viscosity of the reaction mixture increases to render the stirring extremely difficult.

According to the finding of the present inventors, moreover, when MsCl is slowly added dropwise to inhibit the exothermic effect in the above-mentioned Method 2, the chemical conversion rate decreases and the yield decreases.

It was also found that when the reaction is carried out using 2-amino-5-chlorobenzophenone instead of 2-aminobenzophenone according to the above-mentioned Method 2, the objective product could not be purified by recrystallization, since it was an oil, as in Method 1. Similarly, when purification was performed by a step for extraction from the reaction mixture, a concentration step, and a crystallization step, the yield of the hydrochloride crystals was about 60%.

In addition, since dichloromethane used as a solvent in these methods produces a heavy environment burden, it is not a preferable solvent.

Accordingly, an object of the present invention is to provide a method for conveniently producing optically active 2-[(N-benzylprolyl)amino]-5-chlorobenzophenone at a high purity and in a high yield.

The present inventors have conducted intensive studies in an attempt to solve the above-mentioned problems and found that optically active 2-[(N-benzylprolyl)amino]-5-chlorobenzophenone or a salt thereof can be conveniently produced at a high purity and in a high yield by reacting optically active N-benzylproline with 2-amino-5-chlorobenzophenone represented by the formula (2) in acetonitrile in the presence of an amide compound and thionyl chloride, which resulted in the completion of the present invention.

Accordingly, the present invention comprises the following.
[1] A method for producing optically active 2-[(N-benzylprolyl)amino]-5-chlorobenzophenone represented by the formula (3) 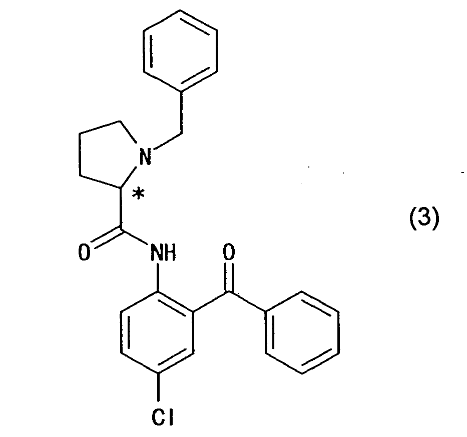 wherein * shows an asymmetric carbon atom and the configuration of the asymmetric carbon atom is S or R, or a salt thereof, which comprises reacting optically active N-benzylproline represented by the formula (1) 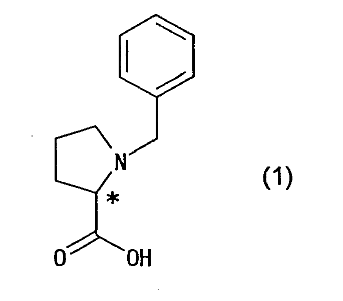 wherein * shows an asymmetric carbon atom and the configuration of the asymmetric carbon atom is S or R, with 2-amino-5-chlorobenzophenone represented by the formula (2) 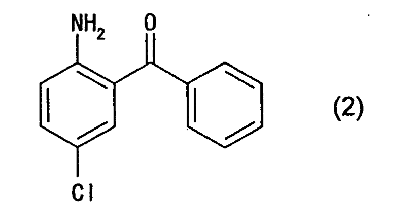 in acetonitrile and in the presence of an amide compound and thionyl chloride.
[2] The method of the above-mentioned [1], wherein the amide compound is N,N-dimethylformamide or N-methyl-2-pyrrolidone.
[3] The method of the above-mentioned [1], wherein the amide compound is N,N-dimethylformamide.
[4] The method of any one of the above-mentioned [1]-[3], wherein the amount of the amide compound to be used is 0.5 to 2 mol per 1 mol of the optically active N-benzylproline represented by the formula (1).
[5] The method of any one of the above-mentioned [1]-[4], wherein the optically active 2-[(N-benzylprolyl)amino]-5-chlorobenzophenone represented by the formula (3) is obtained as a hydrochloride.
[6] The method of any one of the above-mentioned [1]-[5], wherein, in the formula (1) and the formula (3), the configuration of the asymmetric carbon atom is S.
[7] The method of any one of the above-mentioned [1]-[5], wherein, in the formula (1) and the formula (3), the configuration of the asymmetric carbon atom is R.
[8] A method for producing a compound represented by the formula (4) 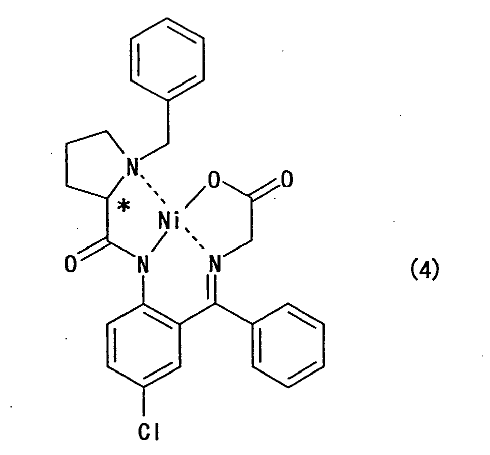 wherein * shows an asymmetric carbon atom and the configuration of the asymmetric carbon atom is S or R, which comprises obtaining optically active 2-[(N-benzylprolyl)amino]-5-chlorobenzophenone represented by the formula (3) or a salt thereof according to the method of any one of the above-mentioned [1]-[5], and reacting the compound of the formula (3) or a salt thereof with glycine and a salt containing Ni²⁺ in the presence of a base.
[9] The method of the above-mentioned [8], wherein, in the formula (1), the formula (3) and the formula (4), the configuration of the asymmetric carbon atom is S.
[10] The method of the above-mentioned [8], wherein, in the formula (1), the formula (3) and the formula (4), the configuration of the asymmetric carbon atom is R.
[11] A hydrochloride of optically active 2-[(N-benzylprolyl)amino]-5-chlorobenzophenone represented by the formula 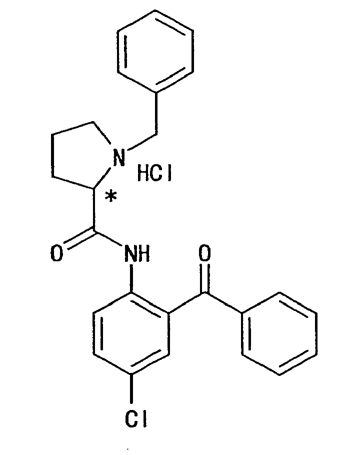
wherein * shows an asymmetric carbon atom and the configuration of the asymmetric carbon atom is S or R.

The method of the present invention is a production method advantageous in the following points.

By reacting optically active N-benzylproline represented by the formula (1) with 2-amino-5-chlorobenzophenone represented by the formula (2) in acetonitrile and in the presence of an amide compound and thionyl chloride, a compound represented by the formula (3) or a salt thereof can be produced at a high purity and in a high yield.

Since HCl generated in the reaction system and a compound represented by the formula (3) form hydrochloride, which is precipitated as crystals, a hydrochloride of the compound represented by the formula (3) can be easily purified by filtering the reaction suspension. Therefore, the extraction step, concentration step and crystallization step can be omitted.

There is no need to use dichloromethane employed in the prior art, which places a large environmental burden. Embodiments of the present invention are explained in detail in the following.

The production method of the present invention is shown by the following scheme. 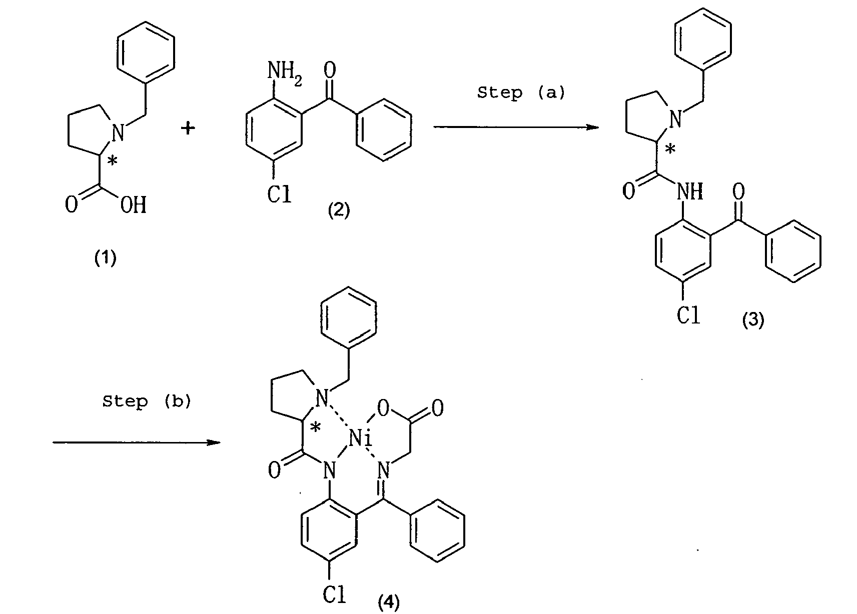 wherein * shows an asymmetric carbon atom and the configuration of the asymmetric carbon atom is S or R.

### Step (a)

In Step (a), optically active N-benzylproline represented by the formula (1) (hereinafter to be referred to as compound (1)) is reacted with 2-amino-5-chlorobenzophenone represented by the formula (2) (hereinafter to be referred to as compound (2)) in acetonitrile and in the presence of an amide compound and thionyl chloride to give optically active 2-[(N-benzylprolyl)amino]-5-chlorobenzophenone represented by the formula (3) (hereinafter to be referred to as compound (3)) or a salt thereof.

In Step (a), acetonitrile is used as a reaction solvent. Using acetonitrile as a reaction solvent in the presence of an amide compound, the objective product at a high purity can be obtained in a high yield. The amount of the solvent to be used is generally 3 to 20 L, preferably 5 to 10 L, relative to 1 kg of compound (1). Other solvent may be mixed with acetonitrile as long as the effect of the invention can be provided.

As the amide compound, N,N-dialkylformamide such as N,N-dimethylformamide; N,N-dialkylacetamide such as N,N-dimethylacetamide; N-alkyl-2-pyrrolidone such as N-methyl-2-pyrrolidone; and the like can be mentioned. Two or more kinds of these amide compounds may be used in a mixture at a suitable ratio. As the amide compound, N,N-dimethylformamide and N-methyl-2-pyrrolidone are preferable, particularly N,N-dimethylformamide is preferable, from the aspect of yield.

The amount of the amide compound to be used is generally 0.5 to 2 mol, preferably 1.0 to 1.2 mol, per 1 mol of compound (1). When the amount of the amide compound is too small or too large, the yield tends to decrease.

While N-benzylproline is poorly soluble in acetonitrile, the solubility of N-benzylproline can be improved by the addition of an amide compound. In addition, the purity of the objective product can be improved by dissolving the byproduct (impurity) in the mother liquor during isolation of the hydrochloride of compound (3) by filtration after completion of the reaction.

The amount of thionyl chloride to be used is generally 1 to 4 mol, preferably 1.1 to 1.5 mol, per 1 mol of compound (1).

The amount of compound (2) to be used is generally 0.5 to 2 mol, preferably 0.9 to 1.0 mol, per 1 mol of compound (1).

For the reaction, it is preferable to add thionyl chloride to an acetonitrile solution containing compound (1) and an amide compound and then add compound (2) to allow a reaction.

While the temperature of the addition of thionyl chloride is not particularly limited, it is generally -78°C to 20°C, preferably -20°C to 10°C. Thionyl chloride is preferably added while stirring the acetonitrile solution, and it is preferable to keep stirring the reaction mixture after the addition of thionyl chloride. While the stirring time after the addition of thionyl chloride is not particularly limited, it is generally 0.1 to 3 hours, preferably 0.5 to 2 hours.

While the reaction temperature of the reaction with compound (2) is not particularly limited, it is generally -78°C to 25°C, preferably -10°C to 25°C. While the reaction time is not particularly limited, it is generally 1 to 24 hours, preferably 4 to 20 hours.

The reaction of compound (1) with compound (2) in the present invention is considered to proceed by a Vilsmeier type reaction in the presence of thionyl chloride and an amide compound (Journal of Polymer Science: Part A: Polymer Chemistry, 24, 701-706, 1986).

Since HCl generated in the reaction system and compound (3) form a hydrochloride, which precipitates as crystals, the hydrochloride of compound (3) can be easily isolated by filtration of the reaction suspension after completion of the reaction.

The hydrochloride of compound (3) can be converted to free compound (3) by treating with a base according to a conventional method. Moreover, the obtained free compound (3) can be converted to other acid addition salt according to a conventional method.

As the salt of compound (3), acid addition salts such as inorganic acid addition salt (e.g., hydrochloride, hydrogen bromide, sulfate, nitrate, phosphate, etc.); organic acid addition salts (e.g., formate, acetate, trifluoroacetate, maleate, tartrate, citrate, fumarate, methanesulfonate, benzenesulfonate, p-toluenesulfonate, etc.); salt with an acidic amino acid (e.g., aspartic acid, glutamic acid, etc.) and the like can be mentioned.

### Step (b)

In Step (b), compound (3) or a salt thereof is reacted with glycine and a salt containing Ni²⁺ in the presence of a base to give a compound represented by the formula (4) (hereinafter to be referred to as compound (4)).

The amount of glycine to be used is generally 1 to 5 mol, preferably 1.2 to 2.0 mol, per 1 mol of compound (3) or a salt thereof.

As the salt containing Ni²⁺, nitrate (Ni(NO₃)₂), halide (e.g., NiCl₂, NiBr₂, etc.), acetate (Ni(OAc)₂), sulfate (NiSO₄) and the like can be mentioned. These salts may be a hydrate, and preferably, Ni(NO₃)₂ or a hydrate thereof.

The amount of the salt containing Ni²⁺ is generally 1 to 5 mol, preferably 1.2 to 2.0 mol, per 1 mol of compound (3) or a salt thereof.

As the base, alkali metal alkoxide (e.g., alkali metal C₁-C₄ alkoxide such as sodium methoxide, sodium ethoxide, sodium isopropoxide, sodium tert-butoxide, potassium tert-butoxide, etc.), alkali metal hydroxide (e.g., sodium hydroxide, potassium hydroxide, etc.) and the like can be mentioned.

The amount of the base to be used is generally 2 to 20 mol, preferably 5 to 10 mol, per 1 mol of compound (3) or a salt thereof.

The reaction of this step is preferably carried out in a solvent that does not inhibit the reaction. As such solvent, alcohol solvents (e.g., methanol, ethanol, isopropanol, t-butanol, etc.), N,N-dimethylformamide, N,N-dimethylacetamide, N-methyl-2-pyrrolidone and the like can be mentioned.

While the amount of the solvent to be used can be appropriately selected according to the kind of the compound, it is generally 2 to 20 L, preferably 4 to 10 L, relative to 1 kg of compound (3).

While the reaction temperature is not particularly limited, it is generally 25°C to 100°C, preferably 40°C to 70°C. While the reaction time is not particularly limited, it is generally 10 minutes to 5 hours, preferably 30 minutes to 2 hours.

The work-up can be performed according to a general method known to those of ordinary skill in the art, and isolation and purification can be performed according to a conventional method appropriately selected as necessary, such as crystallization, recrystallization, distillation, partitioning, silica gel chromatography, preparative HPLC and the like, or according to a combination thereof.

According to method of the present invention, using a compound of the formula (1), wherein the configuration of the asymmetric carbon atom is S, compounds of the formula (3) and the formula (4), wherein the configuration of the asymmetric carbon atom is S, can be obtained. Similarly, using a compound of the formula (1), wherein the configuration of the asymmetric carbon atom is R, compounds of the formula (3) and the formula (4), wherein the configuration of the asymmetric carbon atom is R, can be obtained.

Compound (3) or a salt thereof, and compound (4) produced by the method of the present invention are useful as chiral auxiliary or intermediate for the synthesis of optically active amino acid. For example, compound (4) is reacted with diphenylmethyl halide compound (5) in the presence of a base and the obtained compound (6) is treated with an acid to give optically active diphenylalanine compound (7). After the acid treatment, compound (3) can be recovered as an acid addition salt and can be re-used as a chiral auxiliary. Each step can be performed based on a known method (see Tetrahedron: Asymmetry, Vol. 8, No. 1, pp. 79-83, 1997; J. Org. Chem., Vol. 68, No. 18, pp. 7104-7107, 2003).

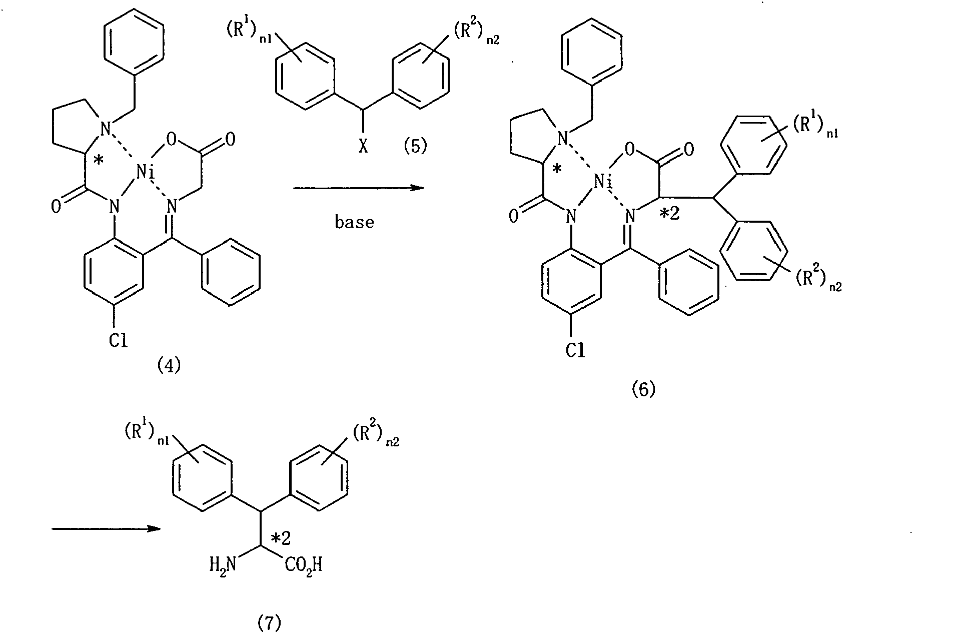

In the above-mentioned scheme, R¹ and R² are each independently a halogen atom (e.g., fluorine atom, chlorine atom, bromine atom, iodine atom), a substituted or unsubstituted alkyl group, a substituted or unsubstituted alkoxy group, a substituted or unsubstituted amino group, a nitro group, a hydroxyl group or a protected hydroxyl group and the like, n1 and n2 are each independently an integer of 0 to 5, X is a halogen atom such as a chlorine atom, a bromine atom and the like, * and *2 show an asymmetric carbon atom, and the configuration of the asymmetric carbon atom shown by * and *2 is (S,S) or (R, R) .

### Examples

Embodiments of the present invention are explained in more detail in the following by referring to Examples, which are not to be construed as limitative.

In the Examples and Reference Examples, the purity and the enantiomeric excess (e.e.) of compound (3) were measured under the following conditions.

### <HPLC conditions-purity>

Column: Inertsil ODS-3 (4.6 x 250 mm)
Eluent:
   A:B=100:0 to 0:100 (0 to 20 min)
   A:B= 0:100 (20 to 40 min)
   A=0.03M KH₂PO₄ aq:CH₃CN=90:10
   B=0.03M KH₂PO₄ aq:CH₃CN=25:75
Flow rate: 1.0 mL/min
Temp.: room temperature
Detector: 210 nm

### <HPLC conditions-chiral>

Column: CHIRALPAK AD (4.6 x 250 mm)
Eluent: hexane:isopropanol=1:1
Flow rate: 1.0 mL/min
Temp.: room temperature
Detector: 220 nm
Retention time:
   (R)-BPC-HCl 5.4 min
   (S)-BPC-HCl 6.3 min

### Reference Example 1

A solution of N-benzyl-L-proline (2.05 g, 10 mmol) in acetonitrile (20 mL) was cooled to -10°C, thionyl chloride (0.875 mL, 1.2 mmol) was slowly added dropwise, and the mixture was stirred for 1 hour. Then, 2-amino-5-chlorobenzophenone (2.09 g, 9 mmol) was added, the temperature was raised to 25°C, and the mixture was stirred for 16 hours. After completion of the reaction, the reaction suspension was filtered, and the filtrated crystals were washed with acetone (3 mL) and vacuum dried to give (S)-2-[(N-benzylprolyl)amino]-5-chlorobenzophenone hydrochloride as white crystals (2.79 g, yield 61%, purity 91%).

### Reference Example 2

To a suspension of N-benzyl-L-proline (10 g, 48.7 mmol) in dichloromethane (20 mL) was added N-methylimidazole (11 mL, 138.0 mmol), and the mixture was cooled to 0°C. Methanesulfonyl chloride (4.5 mL, 58.1 mmol) was added, and 2-amino-5-chlorobenzophenone (10.3 mL, 44.3 mmol) was added. After stirring at room temperature for 30 min, the mixture was refluxed with heating at 46°C for 24 hours. After completion of the reaction, a saturated aqueous ammonium chloride solution (20 mL) was added. The dichloromethane layer was partitioned and concentrated to dryness to give a brown amorphous solid. To this solid were added acetone (71 mL) and concentrated hydrochloric acid (3.9 mL), and the mixture was stirred at room temperature for 1 hour and 30 minutes. The obtained suspension was filtered. The filtrated solid was washed with acetone and vacuum dried to give (S)-2-[(N-benzylprolyl)amino]-5-chlorobenzophenone hydrochloride as pale-brown crystals (12.2 g, yield 60%, purity not less than 99%).

### Example 1

To a solution of N-benzyl-L-proline (38.0 g, 185.3 mmol) in acetonitrile (304 mL) was added N,N-dimethylformamide (14.4 mL, 185.9 mmol) and the mixture was cooled to -10°C. Thionyl chloride (16.2 mL, 222.1 mmol) was slowly added dropwise, and the mixture was stirred for 1 hour. Then, 2-amino-5-chlorobenzophenone (39.42 g, 170.1 mmol) was added, the temperature was raised to 25°C, and the mixture was stirred for 16 hours. After completion of the reaction, the reaction suspension was filtered, and the filtrated crystals were washed with acetone (3 mL) and vacuum dried to give (S)-2-[(N-benzylprolyl)amino]-5-chlorobenzophenone hydrochloride as white crystals (61.3 g, yield 79%, purity not less than 99%, enantiomeric excess not less than 99%e.e.). m.p. 234°C
1H NMR(400MHz, MeOH-d4);1.50-1.59(m, 1H), 1.78-1.90(m, 1H), 2.06-2.16(m, 1H), 2.29-2.38(m, 1H), 3.26-3.3.6(m, 1H), 3.50-3.56(m, 1H), 4.20-4.33(m, 3H), 7.31-7.78(m, 13H) 13C NMR(400MHz, CDCl₃-d1);167.7, 167.5, 150.1, 138.8, 136.9, 134.3, 132.6, 130.6, 130.1, 130.0, 129.4, 128.7, 127.8, 126.6, 125.6, 122.3, 71.1, 59.1, 54.3, 32.2, 22.8

### Example 2

To a solution of nickel nitrate hexahydrate (6.96 g, 23.9 mmol) in methanol (20 mL) were added (*S*)-2-[(N-benzylprolyl)amino]-5-chlorobenzophenone hydrochloride (9.10 g, 20.0 mmol) and glycine (2.25 g, 30.0 mmol), and the mixture was heated to 50°C. 28% sodium methoxide/methanol solution (22 mL) was added, and the mixture was stirred at 60°C for 1 hour. After completion of the reaction, the reaction mixture was added to water to give a reaction suspension. The suspension was filtered and the filtrated solid was vacuum dried to give nickel-(S)-2-[(N-benzylprolyl)amino]-5-chlorobenzophenone-glycine complex as brown crystals (9.76 g, yield 92%).

The results of Reference Example 1, Example 1 and the Examples (Examples 2 and 3 and Reference Examples 3-8), in which a similar operation as in Example 1 was performed except that the solvents and amide compounds described in Table 1 were used, are shown in Table 1. The chemical conversion (yield in the reaction mixture) was measured under the same HPLC conditions as for the purity.

**Table 1**

| | Solvent | Amide compound | Chemical conversion (% ) | Yield (%) | Purity (%) |
|---|---|---|---|---|---|
| Reference Example 1 | acetonitrile | none | 65 | 61 | 91 |
| Example 1 | acetonitrile | DMF | 79 | 79 | >99 |
| Example 2 | acetonitrile | NMP | 81 | 72 | >99 |
| Example 3 | acetonitrile | DMA | 66 | 55 | >99 |
| Reference Example 3 | ethyl acetate | DMF | 67 | 49 | 97 |
| Reference Example 4 | isopropyl acetate | DMF | 49 | - | - |
| Reference Example 5 | THF | DMF | 63 | - | - |
| Reference Example 6 | toluene | DMF | 62 | - | - |
| Reference Example 7 | MTBE | DMF | 43 | - | - |
| Reference Example 8 | acetone | DMF | 35 | - | - |

| | | | | | |
|---|---|---|---|---|---|
| DMF=N,N-dimethylformamide, NMP=N-methyl-2-pyrrolidone, DMA=N,N-dimethylacetamide, THF=tetrahydrofuran, MTBE=methyl tert-butyl ether. | | | | | |

According to the method of the present invention, compound (3) or a salt thereof can be conveniently produced at a high purity and in a high yield. Compound (3) or a salt thereof produced by the method of the present invention is useful as a chiral auxiliary for the synthesis of optically active amino acid.

Obviously, numerous modifications and variations of the present invention are possible in light of the above teachings. It is therefore to be understood that, within the scope of the appended claims, the invention may be practiced otherwise than as specifically described herein.

All patents, patent application publications and other references mentioned above are incorporated in full herein by this reference, the same as if set forth at length.

## Claims

1. A method for producing optically active 2-[(N-benzylprolyl)amino]-5-chlorobenzophenone represented by the formula (3) 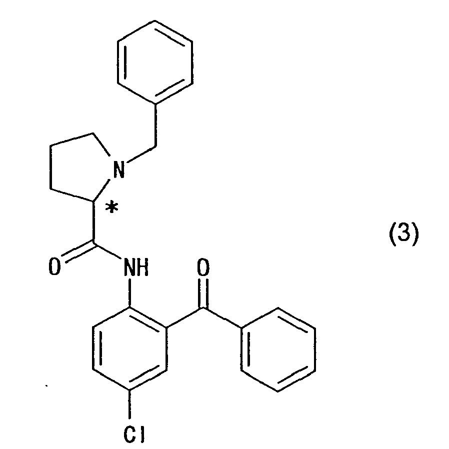 wherein * shows an asymmetric carbon atom and the configuration of the asymmetric carbon atom is S or R, or a salt thereof, which comprises reacting optically active N-benzylproline represented by the formula (1) 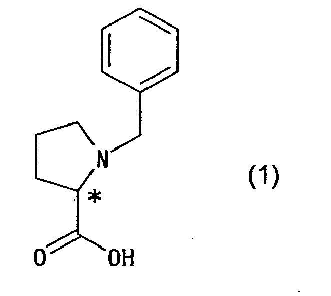 wherein * shows an asymmetric carbon atom and the configuration of the asymmetric carbon atom is S or R, with 2-amino-5-chlorobenzophenone represented by the formula (2) 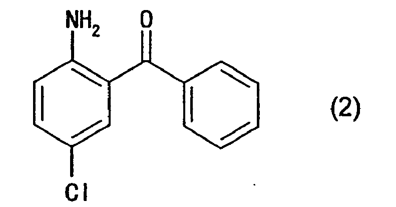 in acetonitrile and in the presence of an amide compound and thionyl chloride.

2. The method of claim 1, wherein the amide compound is N,N-dimethylformamide or N-methyl-2-pyrrolidone.

3. The method of claim 1, wherein the amide compound is N,N-dimethylformamide.

4. The method of any one of claims 1-3, wherein the amount of the amide compound to be used is 0.5 to 2 mol per 1 mol of the optically active N-benzylproline represented by the formula (1).

5. The method of any one of claims 1-4, wherein the optically active 2-[(N-benzylprolyl)amino]-5-chlorobenzophenone represented by the formula (3) is obtained as a hydrochloride.

6. The method of any one of claims 1-5, wherein, in the formula (1) and the formula (3), the configuration of the asymmetric carbon atom is S.

7. The method of any one of claims 1-5, wherein, in the formula (1) and the formula (3), the configuration of the asymmetric carbon atom is R.

8. A method for producing a compound represented by the formula (4) 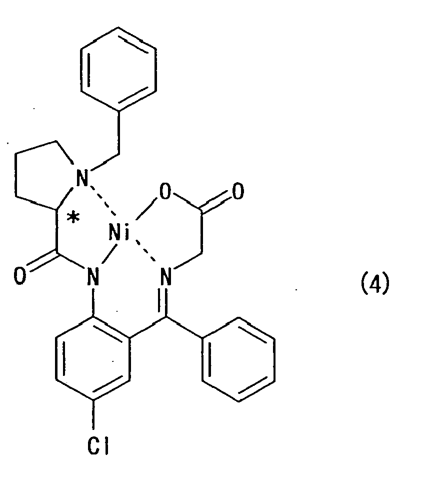 wherein * shows an asymmetric carbon atom and the configuration of the asymmetric carbon atom is S or R, which comprises obtaining optically active 2-[(N-benzylprolyl)amino]-5-chlorobenzophenone represented by the formula (3) or a salt thereof according to the method of any one of claims 1-5, and reacting the compound of the formula (3) or a salt thereof with glycine and a salt containing Ni²⁺ in the presence of a base.

9. The method of claim 8, wherein, in the formula (1), the formula (3) and the formula (4), the configuration of the asymmetric carbon atom is S.

10. The method of claim 8, wherein, in the formula (1), the formula (3) and the formula (4), the configuration of the asymmetric carbon atom is R.

11. A hydrochloride of optically active 2-[(N-benzylprolyl)amino]-5-chlorobenzophenone represented by the formula 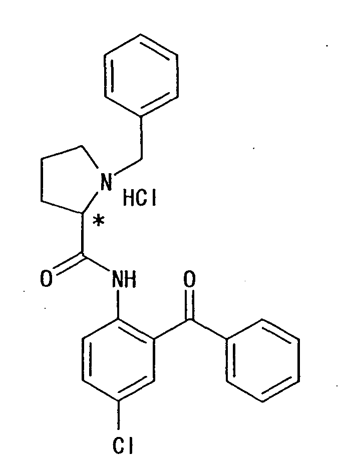 wherein * shows an asymmetric carbon atom and the configuration of the asymmetric carbon atom is S or R.
